Europäisches Patentamt

(19) European Patent Office

Office européen des brevets

(11) Publication number: **0 067 916**
**A1**

## (12) EUROPEAN PATENT APPLICATION

(21) Application number: **81304248.8**

(22) Date of filing: **16.09.81**

(51) Int. Cl.³: **A 61 F 13/16**

(30) Priority: **11.06.81 US 272614**

(43) Date of publication of application:
**29.12.82 Bulletin 82/52**

(84) Designated Contracting States:
**AT BE CH DE FR IT LI LU NL SE**

(71) Applicant: JOHNSON & JOHNSON BABY PRODUCTS
COMPANY
501 George Street
New Brunswick New Jersey 08903(US)

(72) Inventor: Pieniak, Heinz Alfred
12 Nathan Drive North Brunswick
New Jersey 08902(US)

(74) Representative: Jones, Alan John et al,
CARPMAELS & RANSFORD 43 Bloomsbury Square
London, WC1A 2RA(GB)

(54) Quilted diaper and sanitary napkin products.

(57) The present invention relates to a quilted disposable diaper (10) and a quilted sanitary napkin and a method for producing the same. The facing (13) or cover material contains a thermoplastic component either in the fiber or the fiber bonding agent. The facing or cover (13) is heat embossed onto the absorbent batt in a predetermined pattern resulting in the facing or covering adhering to the densified regions (14) making a "quilted" effect.

FIG.1

EP 0 067 916 A1

Croydon Printing Company Ltd.

Quilted Diaper and Sanitary Napkin Products

## Background of the Invention

Disposable diapers have met with increased commercial acceptance in recent years, primarily because of their convenience, as opposed to cloth diapers which need to be laundered once soiled. Many different constructions have been proposed and used, and some have met with widespread commercial success in spite of certain inadequacies in functional properties.

It has long been the desire of the disposable diaper industry to provide a disposable diaper product which keeps the infant dry and prevents leakage, while at the same time handling a full volume discharge of urine. Commonly assigned Mesek et al U. S. Patent 3,612,055 and Burgeni U. S. Patent 3,017,304 disclose several diaper constructions that function extremely well, both in keeping moisture away from an infant's skin and handling a full volume discharge of urine. Similarly, sanitary napkins desirably provide an absorptive product which requires ready absorption of liquid and prevention of leakage.

These functions are accomplished by a multilayer diaper comprising, in order, a fibrous facing layer which is to be brought into contact with the infant's skin, a layer of highly porous, loosely compacted cellulosic fibrous layer integral with the loosely compacted batt and an impervious backing sheet adhered to the densified

JBP 207

layer through the interface therebetween. The facing layer is of porous construction and its fibers have less wettability for water than the fibers of the loosely compacted batt, resulting in a tendency for liquid to flow from the facing web into the batt. The densified fibrous layer has a smaller average pore size than the loosely compacted batt, resulting in a tendency for liquid to flow preferentially from the batt into the underlying densified layer, rather than to other areas of the batt, thus tending to restrict wetting in the batt to an area of moderate size. Liquid flowing into the densified layer tends to spread laterally because of its wicking action and liquid which might pass through the densified layer during discharge (when flow is rapid) is held back by the impervious backing sheet for sufficient time to permit absorption to take place. Liquid in excess of the absorptive capacity of the densified layer is forced back from the impervious layer into the dry portion of the loosely compacted batt, thus utilizing the additional absorptive capacity therein. The sanitary napkin product is similarly structured in that there generally is a moisture-impervious barrier surrounding the underside and the side portions of the absorbent batt, then the entire batt is wrapped in a cover which, for the purposes discussed herein, will be called a facing layer.

There are basically three different types of absorbent batts, viz., those wherein the paper-like, densified layer extends continuously over a given area of the loosely compacted batt; those wherein the paper-like densified layer is discontinuous and arranged in a preselected geometric pattern; and those having no paper-like densified area or skin. The advantage of

JBP 207

the absorbent structures having a densified skin is the ability to provide directionalized fluid flow, i.e. the fluid tends to flow in the direction of the paper-like densified portions as opposed to flowing into the loosely compacted portions of the absorbent batt. Thus, by utilizing an absorbent batt wherein the spaced densified portions extend in a lengthwise direction, the fluid flows preferentially along the densified portions to spread out longitudinally within the densified layer before spreading laterally. In addition to, or in place of the paper-like densified skin, densified regions densifying the entire thickness of the batt may be formed. Generally such densified regions are formed by embossing. These densified regions described in U. S. Patent 3,938,522, Repke, promote integrity of the absorbent batt and assist wicking of liquid from one area to another in the fibrous structure. Absorbent batts, having spaced densified regions, provide increased ability to control and direct the flow of liquid, as opposed to a continuous paper-like, densified layer wherein the fluid flow is substantially equal in all directions. Furthermore, because the spaced densified regions of the batt are separated by loosely compacted batt portions having limited wickability, the linear flow rate in the densified portions is far greater than the rate of lateral spread with the result that fluid often reaches the ends of the densified portions before it spreads outwardly into previously unwet portions of the absorbent structure.

It has also been found that when the synthetic nonwoven fabrics such as polyester, polyethylene, polypropylene, rayon and the like, are used as the facing layer in the diaper, the absorbent batt tends to break apart

P 207

when in use. Attempts have been made to improve the integrity of the batt by wrapping the batt in tissue or by applying adhesives to one surface of the batt. These procedures can be cumbersome and expensive.

Summary of the Invention

The present invention provides a multilayer diaper comprising a porous facing layer adapted to be positioned adjacent an infant. The facing layer includes a thermoplastic component. Adjacent the facing layer, in juxtaposition thereto, is a highly porous, loosely compacted, cellulosic fibrous batt. The diaper further contains a plurality of spaced densified compacted cellulosic regions integral with the loosely compacted batt, at least some of the densified regions extending through the entire cross-sectional thickness of the batt. The facing layer is adhered to the batt only in the densified regions whereby the diaper is stabilized and reinforced while maintaining a soft flexible surface to be positioned adjacent the infant. The diaper also has a water-impervious backing sheet in face-to-face juxtaposition to the batt on the side thereof opposite the facing layer.

The present invention also provides a sanitary napkin product comprising an absorbent batt generally of rectangular shape, surrounded by a nonwoven fabric cover or facing layer containing a thermoplastic substance. The facing layer is adhered to densified regions placed in the absorbent batt whereby the sanitary napkin is stabilized and reinforced while maintaining a soft flexible surface to be positioned adjacent the infant. Preferably, the sanitary napkin has a moisture-impermeable

JBP 207

layer between the facing layer and the batts placed so as to substantially cover the side of the batt opposite that facing the wearer and coming up around each of the sides. The cover or facing layer generally extends at each end of the batt so as to provide tabs which may be used for securing the napkin to a belt device or wearing apparel.

The present invention also provides a method of producing an integral stabilized multilayer disposable diaper by providing a nonwoven fabric facing layer containing a thermoplastic substance and a loosely compacted cellulosic batt. The facing layer is then adhered to densified regions placed in the batt either prior to or simultaneously with the adhering step. The lightly compacted cellulosic batt contains at least 3% moisture. The facing layer is adhered to the batt by compaction or embossing with sufficient heat to plasticize the thermoplastic substance. A water-impervious backing sheet is provided in face-to-face juxtaposition to the batt on the side opposite the facing layer.

The embossing lamination can be of any desirable pattern such as parallel lines, crossed lines providing shapes such as a diamond shape, rectangular shapes, circular shapes, etc. It is not necessary that the densified regions having the facing adhered thereto extend completely to the edge of the absorbent batt. In other words, the facing layer is laminated to the densified regions in the absorbent batt in at least the central portion of the diaper and any other desirable parts of the diaper structure. The resultant disposable diaper is in effect "quilted." In other words, between the densified regions there are pillow-like areas of

JBP 207

loosely compacted fluff.

## Brief Description of the Drawings

FIGURE 1 is a perspective view of a disposable diaper of the present invention;

FIGURE 2 is a perspective view of the disposable diaper of Figure 1 when applied about an infant;

FIGURE 3 is a plan view of another embodiment of a disposable diaper, the invention with a portion broken away to show interior detail;

FIGURE 4 is a plan view of still another embodiment of the present invention with a portion broken away to show interior detail;

FIGURE 5 is a sectional view on an enlarged scale taken along line 5—5 in Figure 3;

FIGURE 6 is a plan view of a still further embodiment of a disposable diaper of the present invention with a portion broken away to show interior detail;

FIGURE 7 is a perspective view of a sanitary napkin of the present invention;

FIGURE 8 is a cross-sectional view on an enlarged scale taken along line 8—8 in Figure 7; and

FIGURE 9 is a plan view of another embodiment of a sanitary napkin.

JBP 207

Detailed Description of the Present Invention

While this invention is susceptible of embodiment in many different forms, there is shown in the drawings and will herein be described in detail, preferred embodiments of the invention and modifications thereof, with the understanding that the present disclosure is to be considered as an exemplification of the principles of the invention and is not intended to limit the invention to the embodiments illustrated.

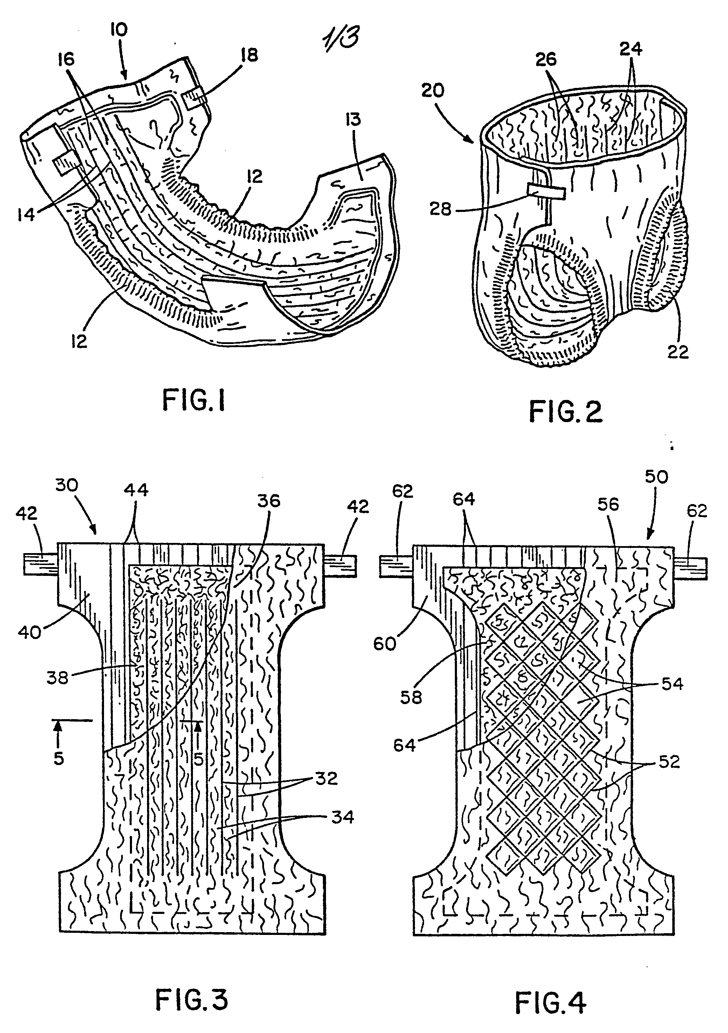

Referring to the drawings and particularly to Figures 1 and 2, in Figure 1 the diaper assembly 10, when in an open position, comprises an elastic leg band area 12, densified regions 14 wherein the facing 13 is adhered to the densified regions 14 creating pillow-like convolutions 16. Tape tab 18 is placed at one end of the diaper for fastening the diaper about the infant. In Figure 2 the same diaper structure 20, as in Figure 1, is shown as it would be placed upon an infant. The densified regions 24 create the pillow-like convolutions 26. Here again, an elastic leg band 22 secures the diaper about the leg. Tape tabs 28 secure the diaper about the waist.

In Figure 3 a flat diaper structure 30 is illustrated. The densified regions 32 are in parallel linear form and create pillow-like convolutions 34. In the breakaway portion, the absorbent 38 can be seen superimposed upon the backing 40 and the facing layer 36 is superimposed on the other side of the absorbent batt 38. The facing layer 36 and backing sheet 40 are secured to each other in the margins by glue lines 44. The glue lines 44 also secure the absorbent batt to the

JBP 207

0067916

backing sheet.

Referring now to Figure 4, diaper structure 50 comprises a shaped absorbent batt 58 and the facing 56 and the backing 60 are of similar contour but larger than the absorbent batt, thus creating a margin surrounding the absorbent batt. The densified regions 52 are in a diamond-shape pattern and create pillow-like convolutions 54, thus giving a quilted effect. Glue lines 64 secure the facing and backing to each other in the margins and the absorbent batt 58 to the backing sheet 60. The tape tabs 62 secure the diaper structure about the infant.

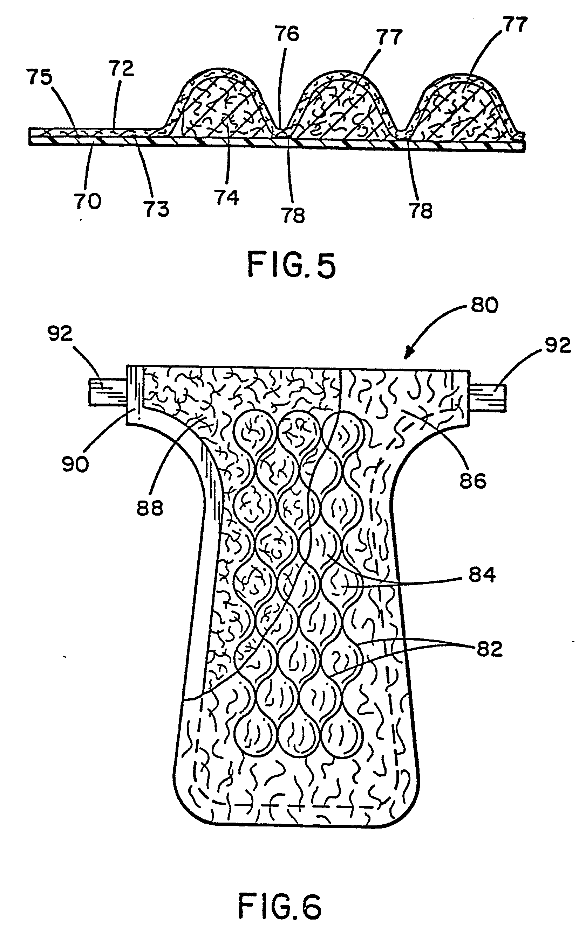

In Figure 5 a cross-section view of a portion of the diaper structure 30 of Figure 3 is shown. The backing sheet 70 is secured in the margin by glue lines 73 and 75 to the facing 72. The absorbent batt 74 is densified in regions 78 in what appear to be valleys 76. The facing 72 is adhered to the densified regions 78. This results in the creation of loosely compacted areas 77, thus creating a quilt-like structure.

Referring now to Figure 6, a T-shaped diaper 80 is shown. The densified regions 82 are in a continuous pattern which create soft lofty areas 84, giving a quilt-like appearance. The facing sheet 86 is adhered to the densified regions 82. In the breakaway portion the absorbent batt 88 is shown in relation to the backing sheet 90. Tape tabs 92 function to secure the diaper about the infant.

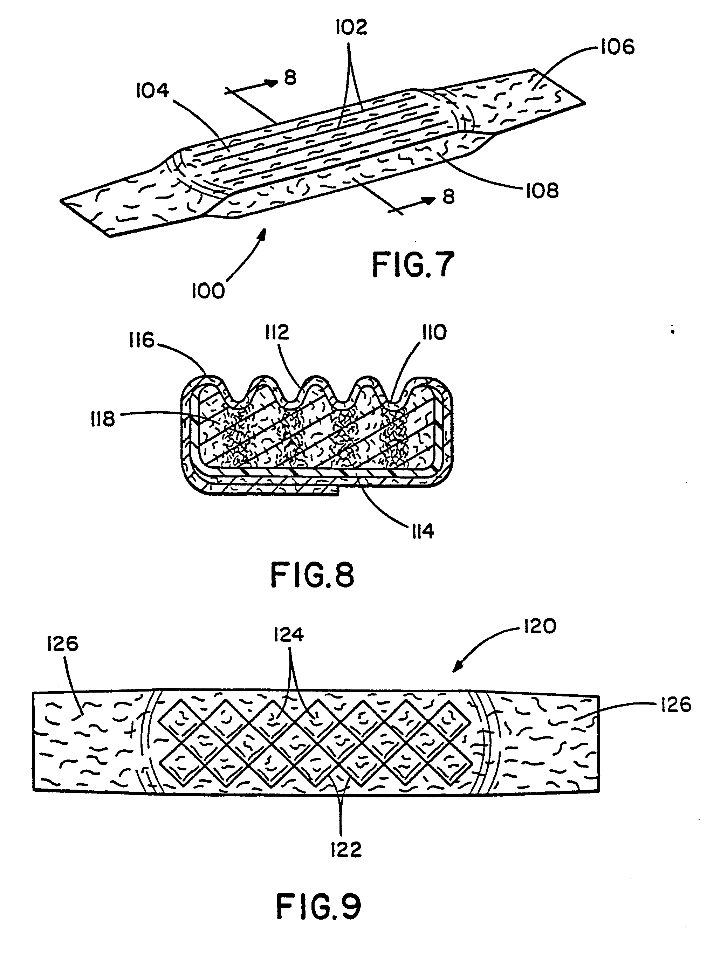

Referring now to Figure 7, a sanitary napkin 100 is shown. The densified regions 102 are in a line pattern which

JBP 207

create soft, lofty areas 104 between the parallel lines. The facing sheet 106 is adhered to the densified regions 102 of the absorbent batt 108.

In Figure 8, a cross-sectional view of the sanitary napkin of Figure 7 is shown. The densified regions 110 wherein the cover 116 is adhered to the absorbent batt 118 create lofty areas 112. The densified regions 110 readily accept and transport liquid, assisting in keeping the lofty regions 112 substantially dry. A moisture-impermeable layer 114 lies between the facing layer 116 and the absorbent batt 118.

Figure 9 illustrates a plan view of a sanitary napkin 120 wherein the embossed densified regions 122 are in a diamond pattern. These densified regions create lofty areas 124. The exterior cover of the sanitary napkin, i.e., the facing layer, is adhered to the loosely compacted cellulosic batt below it in the densified regions. The sanitary napkin 120 has extensions of the facing layer 126 which provide attachment tabs.

Suitable fibrous structures for making the absorbent batts used in this invention are made from short cellulosic fibers, such as wood pulp fibers or cotton linters, obtained by the grinding or comminution of compacted wood pulp fibers or cotton linters. The batts are initially formed by airblowing the slightly moist cellulosic fibers onto a support at a total weight of about 2 to about 20 oz./sq.yd. and then subjecting the airblown fibers to compression. The compacted cellulosic material is preferably at a moisture content of 3 - 10% by weight before being subjected to the grinding operation so that the fibers produced by grinding have sufficient moisture to have the capability of developing some interfiber hydrogen bonds which provide coherence to the body of the batt.

Once the batt is formed, the facing layer is placed in juxtaposition to the batt covering one surface area of the batt and the two layers are laminated in preselected regions by application of heat and pressure. The heat is sufficient to render the thermoplastic substance in the facing layer plastic. Thus, the facing layer is adhered to the batt in the regions where heat and pressure are applied. Suitable pressures range from at least about 40 psi to about 100 psi or more.

In one embodiment, prior to the formation of the densified regions, a dense compacted paper-like layer or skin is formed on one surface of the cellulosic batt. This skin is prepared by moistening the surface of the cellulosic batt with a fine spray of water and then subjecting the moistened batt to pressure. The formation of the densified skin on the cellulosic batt is believed to be due to the formation of hydrogen bonds between contracting moistened fibers similar to the bonds between the fibers in paper. By the proper selection of the amount of moisture applied to the surface of the batt and by the proper selection of the degree of compression imposed, the properties of the densified skin may be varied as desired. The thickness, density, strength and other characteristics of the densified paper-like skin will depend on the uniformity by which the moisture is applied, the depth to which it penetrates and the degree to which the fibers are compressed. After compression of the cellulosic batt to form the paper-like skin, the facing layer is placed in position and densified regions in a prescribed pattern are formed in the manner heretofore set forth causing the facing layer to adhere to the densified regions. Preferably,

IBP 207

the facing layer is embossed onto the cellulosic batt.
Depending on the thermoplastic substance in the facing
layer, heat to a predetermined degree is applied during
the embossing of the facing layer to effect adhering
the facing layer to the densified regions.  Care is
taken not to further compact the nondensified regions.

The least wettable of the fibrous elements of the diaper
or sanitary napkin of the present invention is the facing
layer.  This facing layer is a nonwoven fabric containing
either thermoplastic fibers or having thermoplastic
elements in the bonding agent used to form the nonwoven
fabric.  Suitable fibers include polyester, polypropylene,
polyethylene, rayon or the like.  By providing a thermoplastic element in the facing layer fabric, it is
possible to adhere the facing layer to the densified
regions in the diaper structure by the application of
heat and pressure.  It is desirable to have the facing
layer water repellent so as to assist in keeping the
infant dry.  However, the facing layer also must be
penetrated by the urine at the time of voiding by the
infant.  Thus, the facing layer is receptive to penetration by urine, but remains less wettable than the batt.

A useful parameter of wettability is a liquid-fiber
contact angle of the individual fibers of the layer, the
contact angle approaching 90° for fibers which are
difficultly wettable, exceeding 90° for fibers which
are highly water repellent, and approaching 0 for fibers
which are easily wettable by water.

In any particular facing layer, the liquid-fiber contact
angle for individual fibers may vary considerably because
of unevenness of distribution of the water-repellent
bonding agent and unevenness of the distribution of any wetting agent that might have been added. Nevertheless, a liquid-fiber contact angle between about 30° and about 60° for more of the individual fibers in a random selection provides suitable wettability in the facing layer and a liquid-fiber contact angle between about 40° and about 50° is preferable.

The body of the batt is substantially more wettable than the facing layer and tends to draw liquid away from the facing layer. The individual fibers of the batt are extremely wettable, generally have liquid-fiber contact angles below about 15° and approach 0 in the optimum embodiment. The wickability, or preferential absorptivity of the body of the batt for water is limited, however, by its low density which results in a large effective capillary radius for the capillaries between adjacent fibers.

The pressure causing a liquid to enter a cylindrical capillary is expressed by the equation:

$$P = \frac{2\gamma \cos \theta}{r}$$

where  P is the capillary pressure,
       $\gamma$ is the surface tension of the liquid,
       $\theta$ is the liquid-fiber contact angle, and
       r is the capillary radius.

With a given liquid, the pressure (capillary force) increases with the cosine of the liquid-fiber contact angle (reaching a maximum where the angle is 0), and increases with narrower capillary radii so that narrower capillaries will draw liquid from wider ones.

The relative wickability between the facing layer and the body of the batt is affected by both the relative densities of the layers and the relative wettability of the individual fibers in each layer. The facing layer is sometimes more dense than the body of the batt, but even then the individual fibers of the batt have substantially smaller liquid-fiber contact angles than those of the facing layer, overcoming the density difference and providing a substantial overall increase in capillary pressure to absorb liquid into the body of the batt.

In the present structure wherein the facing layer is adhered to the absorbent batt in a prescribed pattern of densified regions, the liquid penetrates the facing layer rapidly at these densified region areas. Due to the density and thus higher capillary pressure in the densified regions, the liquid is rapidly transported from the void area into other areas of the absorbent batt structure. As the less dense areas gradually absorb the liquid, the wearer's skin is maintained dry due to the pillow-like convolutions of the less densified areas. In other words, in the quilt-like structure, the densified regions to which the facing layer is adhered act as highways to conduct the liquid away from the void zone to other portions of the diaper structure. On the other hand, the light fluffy non-densified regions act as pillows surrounding these highways and thus keep the wearer's skin away from the wet area.

In one embodiment, a cellulosic batt of wood pulp fibers is formed by air-laying the fibers on a foraminous continuously moving belt. Water is sprayed on the under surface of the batt to moisten the surface thereof. The batt is then subjected to compression between a pair of rolls to form a paper-like densified layer. A polyester nonwoven fabric with a weight of 0.7 oz/sq.yd. is placed

JBP 207

on the dry surface of the cellulosic batt. The two layers are embossed at a temperature of 235°F at 40 psi in a pattern of 5 longitudinal lines extending most of the length of the batt leaving unembossed portions of about one, inch at each end of the line. After embossing and thus adhering the polyester fabric to the cellulosic batt in the embossed regions, a polyethylene backing sheet is applied to the side of the densified paper-like layer of the cellulosic batt. The multilayered structure is laminated in the margins beyond the batt with adhesive. Tape tabs are then applied at one end of the diaper for use to secure the diaper about an infant.

In another embodiment, the same process followed in the previous embodiment is used but the unwoven fabric is a polypropylene fabric. Substantially the same conditions are used.

In a still further embodiment, a cellulosic batt is formed as before. The batt is then placed on the polyethylene backing having glue lines on the side of the backing sheet toward the batt. Next the facing layer is placed on the opposite side of the batt from the backing sheet. The facing layer is a nonwoven rayon fabric having a thermoplastic bonding agent. In other words, the bonding agent used when the rayon fabric was formed is thermoplastic. After the facing layer is placed on the batt, the entire structure is subjected to lamination by embossing rolls which place five longitudinal lines on the central region of the diaper laminating the facing to the batt and laminating rolls which laminate the facing and the backing in the margins.

Similarly, a sanitary napkin is constructed by forming the cellulosic batt, placing the moisture-impermeable film such as polyethylene film, on three sides of the
batt leaving a large surface area open. This partially
wrapped cellulosic batt is then completely wrapped in
the facing layer using, for instance, a polyester,
nonwoven fabric. A predetermined pattern is heat embossed
on the surface area not encompassed by the liquid
impervious film so as to laminate the facing to the batt
to provide pillow-like convolutions of the less densified
areas. The facing layer extends beyond the length of the
cellulosic fibrous batt providing attachment tabs.

JBP 207

Claims:

1. An absorbent product comprising a soft, flexible porous facing layer adapted to be positioned adjacent the wearer's skin, said facing layer including a thermoplastic component; a highly porous, loosely compacted cellulosic fibrous batt at least one of which surface is covered by said facing layer; a plurality of spaced, densified compacted cellulosic regions integral with said loosely compacted batt at least some of said densified regions extending through substantially the entire cross-sectional thickness of the batt, said facing layer being adhered to said batt only in the densified regions whereby said absorbent structure is stabilized and reinforced.

2. The absorbent product of Claim 1 wherein the product is a sanitary napkin.

3. The absorbent product of Claim 1 wherein the product is a diaper.

4. The absorbent product of/Claims1/wherein said porous facing layer is a nonwoven fabric selected from the group consisting of polyester, polypropylene, polyethylene and rayon.

*any one of to 3*

5. The absorbent product of Claim 4 wherein said thermoplastic component is the fibers of the nonwoven fabric.

6. The absorbent product of Claim 4 wherein said thermoplastic component is in the bonding agent of said nonwoven fabric.

7. The absorbent product of Claims 1 any one of to 6 wherein said plurality of spaced, densified compacted cellulosic regions are embossed longitudinal lines extending most of the length of said batt.

8. The absorbent product of Claims 1 any one of to 6 wherein said plurality of spaced, densified compacted cellulosic regions are embossed intersecting lines which form a diamond pattern.

9. The absorbent product of Claims 1 any one of to 8 wherein said batt contains a paper-like densified layer integral with said batt.

10. A multilayer disposable diaper comprising a soft, flexible porous facing layer adapted to be positioned adjacent an infant, said facing layer including a thermoplastic component; a highly porous, loosely compacted, cellulosic fibrous batt in face-to-face juxtaposition to said facing layer; a plurality of spaced densified compacted cellulosic regions integral with said loosely compacted batt, at least some of said densified regions extending through the entire cross-sectional thickness of the batt, said facing layer being adhered to said batt only in the densified regions whereby said diaper is stabilized and reinforced; and a water-impervious backing sheet in face-to-face juxtaposition to said batt on the side thereof opposite from said facing layer.

11. The diaper of Claim 10 wherein said porous facing layer is a nonwoven fabric selected from the group consisting of polyester, polypropylene, polyethylene and rayon.

JBP 207

12. The diaper of Claim 11 wherein said thermoplastic component is the fibers of the nonwoven fabric.

13. The diaper of Claim 11 wherein said thermoplastic component is in the bonding agent of said nonwoven fabric.

14. The diaper of/Claims 10 any one of to 13 wherein said plurality of spaced densified compacted cellulosic regions are embossed longitudinal lines extending most of the length of said batt.

15. The diaper of/Claims 10 any one of to 13 wherein said plurality of spaced densified compacted cellulosic regions are embossed intersecting lines which form a diamond pattern.

16. The diaper of/Claims 10 any one of to 15 wherein said batt contains a paper-like densified layer integral with said batt.

17. A disposable sanitary napkin comprising a soft, flexible porous facing layer adapted to be positioned adjacent the wearer's skin, said facing layer including a thermoplastic component; a highly porous, loosely compacted cellulosic batt encompassed by said porous facing layer; and a plurality of spaced, densified compacted cellulosic regions integral with said loosely compacted batt, at least some of said densified regions extending substantially through the entire cross-sectional thickness of the batt, said facing layer being adhered to said batt only in the densified region whereby said sanitary napkin is stabilized and reinforced.

18. The sanitary napkin of Claim 17 wherein said porous facing layer is a nonwoven fabric selected from the group consisting of polyester, polypropylene, polyethylene and

JBP 207

rayon.

19. The sanitary napkin of Claim 18 wherein said thermoplastic component is the fibers of the nonwoven fabric.

20. The sanitary napkin of Claim 18 wherein said thermoplastic component is in the bonding agent of said nonwoven fabric.

21. The sanitary napkin of /Claims 17/ any one of to 20 wherein said plurality of spaced densified compacted cellulosic regions are embossed longitudinal lines extending most of the length of said batt and placed on the surface which faces the wearer.

22. The sanitary napkin of /Claims 17/ any one of to 20 wherein said plurality of spaced densified compacted cellulosic regions are embossed intersecting lines which form a diamond pattern and which are on the surface which faces the wearer.

23. The sanitary napkin of /Claims 17/ any one of to 22 wherein said batt contains a paper-like densified layer integral with said batt and on the surface of said batt opposite the side facing the wearer.

24. A method of producing an integral stabilized multi-layer absorbent product comprising providing a loosely compacted cellulosic batt having relatively low cohesive strength, providing a soft, flexible porous facing layer adapted to cover at least one surface of said batt and to be positioned adjacent the wearer's skin and including a thermoplastic component, embossing said batt and said facing layer to provide a laminate whereby a plurality of

BP 207

spaced densified regions are formed in said batt and said facing is adhered to said regions, and placing a water-impervious backing sheet on the side opposite the cellulosic batt where said facing layer is adhered.

25. The method of Claim 24 wherein said embossing step is performed at a pressure of at least 40 psi and a temperature sufficient to render the thermoplastic component plastic.

26. A method of producing an integral stabilized multi-layer disposable diaper comprising forming a loosely compacted cellulosic batt having low cohesive strength, forming a paper-like densified region integral with said batt, laminating a soft, flexible porous facing layer including a thermoplastic component by heat embossing and adhering a water-impervious backing sheet to the resulting laminate on the side opposite said facing layer.

27. A method of producing a stabilized, multi-layer sanitary napkin comprising forming a loosely compacted cellulosic batt having low cohesive strength, placing a water-impervious backing sheet on one side of said cellulosic batt covering at least the opposite side of said cellulosic batt with a soft, flexible porous facing layer including a thermoplastic component and heat embossing and adhering the facing layer to said cellulosic batt.

JBP 207

1/3

FIG.1

FIG.2

FIG.3

FIG.4

75 72 76 77 77

70 73 74 78 78

**FIG.5**

92 80 92

90 88 86

84

82

**FIG.6**

FIG.7

FIG.8

FIG.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | GB-A- 794 500 (CHICOPEE MANUFACTURING CORP.)<br><br>* page 2, line 47-81; figures * | 1-4,6-8,10,11,13-15,17,18,20-22,24 | A 61 F 13/16 |
| Y | | 5,12,19 | |
| Y | GB-A-2 034 604 (KAO SOAP)<br><br>* claims 1-3; figures * | 5,12,19 | |
| A | US-A-3 707 430 (COSTANZA) | 1,10,17,24 | |
| A | US-A-3 881 490 (WHITEHEAD) | 1,10,17 | TECHNICAL FIELDS SEARCHED (Int. Cl. 3)<br><br>A 61 F<br>A 41 B |

The present search report has been drawn up for all claims

| Place of search | Date of completion of the search | Examiner |
|---|---|---|
| THE HAGUE | 14-09-1982 | STEENBAKKER J. |